# EUROPEAN PATENT SPECIFICATION

(11) **EP 0 217 327 B1**
(45) Date of publication and mention of the grant of the patent: **30.06.1993**
(21) Application number: 86113349.4
(22) Date of filing: 29.09.1986
(51) Int. Cl.: C12N 15/12, C12N 1/21, C12N 5/00

(54) **DNA Sequence encoding variant elongation factor 2**
Für eine Variante des Elongationsfaktors 2 kodierende DNS-Sequenz
Séquence d'ADN codant pour une variante du facteur d'élongation 2

(30) Priority: 30.09.1985 JP 219545/85
(43) Date of publication of application: 08.04.1987
(73) Proprietor: SUMITOMO PHARMACEUTICALS COMPANY, LIMITED, Osaka-shi, Osaka-fu 541 (JP)
(72) Inventor: Uchida, Tsuyoshi, Toyonaka-shi Osaka-fu (JP)
(74) Representative: VOSSIUS & PARTNER

(56) References cited:
- EP-A- 0 133 671
- CHEMICAL ABSTRACTS, vol. 102, no. 3, 1st April 1985, page 161, abstract no. 107151b, Columbus, Ohio, US; Y. KANEDA: "Gene mapping of human EF-2"
- PROC. NATL. ACAD. SCI. USA, vol. 81, May 1984, pages 3158-3162; Y. KANEDA et al.: "Chromosomal assignment of the gene for human elongation factor 2"
- PROC. NATL. ACAD. SCI. USA, vol. 83, July 1986, pages 4978-4982; K. KOHNO et al.: "Amino acid sequence of mammalian elongation factor 2 deduced from the cDNA sequence: Homology with GTP-binding proteins"
- J. Biol. Chem. vol. 249 (1974), pp. 5088-5093

## Description

The present invention relates to a DNA sequence encoding elongation factor 2 associated with an elongation of a polypeptide chain and its mutants, and use of the latter as a selective marker. The elongation factor 2 will hereinafter be referred to as EF-2.

EF-2 is essential for translocation of peptidyl-tRNA from A-site to P-site on the ribosome, said translocation being inevitable for biosynthesis of proteins in eucaryotic cells. EF-2 is inactivated through the formation of a covalent bond with ADP ribose derived from NAD by the action of diphtheria toxin or an exotoxin of Pseudomonas aeruginosa. Accordingly, cells exposed to such toxin is likely to die because EF-2 is inactivated and, therefore, protein synthesis is prohibited.

It has already been known that EF-2 protein in rat contains the following partial amino acid sequence which constitutes the proximal region of the binding site at which the protein forms a covalent bond with ADP ribose: 
Phe-Asp-Val-His-Asp-Val-Thr-Leu-His-Ala-
Asp-Ala-Ile-X-Arg
wherein X represents histidine residue which undergoes modification to form "diphthamide structure" with which ADP ribose forms a covalent bond. The "diphthamide structure" has recently been reported in detail. It has been also known that the human EF-2 gene locates on the 19th chromosome (see Chemical Abstracts, Vol. 102, No. 3, page 161, abstract no. 1071516; Kaneda, Y., Nippon Islaikai Zasshi, Vol. 92, No. 7, 1082-1088, 1984).

The inventor of the present invention previously succeeded in isolating EF-2 from rat liver and determined the base sequence of 19 amino acid residues at N-terminal, which is as follows: 
Val-Asn-Phe-Thr-Val-Asp-Gln-Ile-Arg-Ala-
Ile-Met-Asp-Lys-Lys-Ala-Asn-Ile-Arg
The inventor also determined that C-terminal of EF-2 was leucine.

With above information, the inventor has made an extensive study on EF-2 protein and succeeded in determining a full length of the nucleotide sequence of cDNA encoding said protein essential for survival of living cells and determining the primary structure of the protein.

On the other hand, the inventor succeeded in isolating from chinese hamster ovary (CHO) cells a mutant cell which acquired a resistance to diphtheria toxin and P. aeruginosa exotoxin. A variant EF-2 which is responsible for the acquired resistance of the CHO cell as well as cDNA encoding such variant EF-2 were also obtained on the basis of the mutant cell. Among them, the cDNA coding for the variant EF-2 has been found to be especially useful as a selective marker.

Accordingly, the invention provide a DNA sequence coding for EF-2 or variant EF-2 responsible for resistance against diphtheria toxin and P. aeruginosa exotoxin. The invention also provides a replicable plasmid vector which harbours said DNA encoding variant EF-2.

The invention further provides a microorganism or cell culture transformed with the plasmid vector harbouring said variant EF-2-encoding DNA.

In the accompanying drawings:
Fig. 1 shows the DNA sequence of plasmid pHEW 1 (about 3 kb) which harbours the full length EF-2 gene and the deduced amino acid sequence thereof. The domain ranging from the start codon ATG at 78 position to the stop codon TAG at 2652 position encodes the naturally occurring EF-2 protein.
Fig. 2 shows the DNA sequence of plasmid pHED 1 which carries variant EF-2 gene and deduced amino acid sequence thereof. The domain ranging from the start codon ATG at 78 position to the stop codon at 2652 position encodes variant EF-2 which endows cells with ability to resist against diphtheria toxin and P. aeruginosa exotoxin.
Fig. 3 is a restriction map of pHEW 1.
Fig. 4 is a restriction map of plasmid pRE 2 which harbours rat EF-2-encoding DNA. Fig. 4 also shows fragments A and B employed as a probe in detecting said DNA.
Fig. 5 shows an amino acid sequence constituting proximal region of the binding site at which EF-2 protein binds to ADP ribose. The sequence of a synthetic oligonucleotide employed as a probe is also provided in this figure.

As previously stated, the DNA encoding variant EF-2 of the invention is particularly useful as a selective marker. For instance, when an animal cell culture is transformed with an expression plasmid vector harbouring said variant DNA and cultured in the presence of diphtheria toxin or P. aeruginosa exotoxin, only cells which acquired said variant DNA can remain alive. Accordingly, where a gene of interest which contains no selectable marker is incorporated into an arbitrary cell culture after ligated to the variant DNA, cells which acquired the gene of interest can be efficiency selected on the basis of the resistance to the toxins.

Genes designated pSV 2-gpt and pSV 2-neo are hitherto known to be useful as a selective marker and can be used for the selection purpose as mentioned above. However, the variant DNA of the invention has a wide spectrum of recipient cells and requires less cultivation time for selection as compared with these known markers.

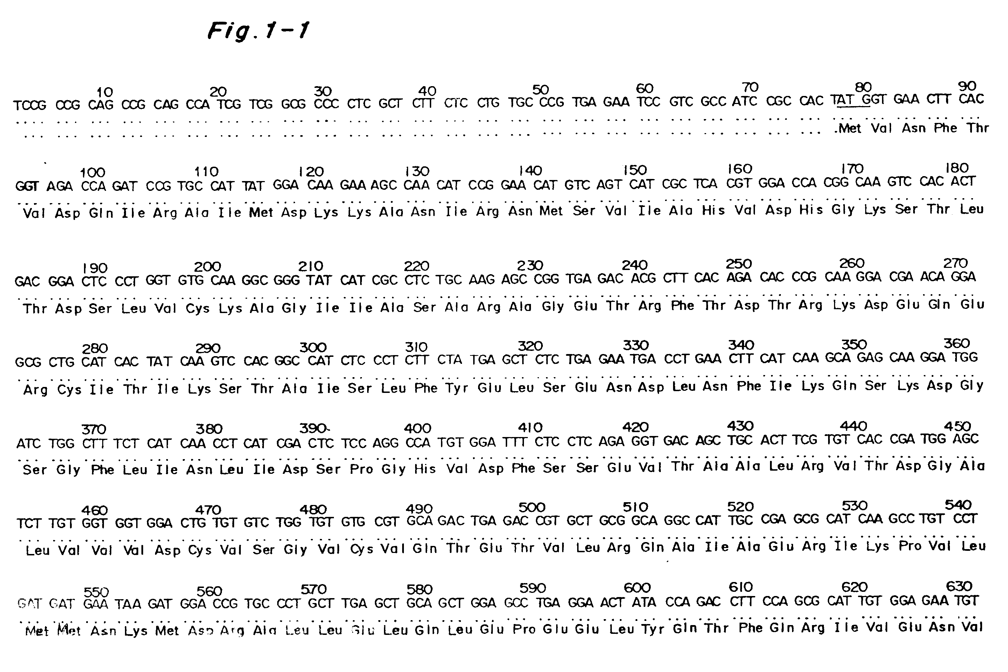

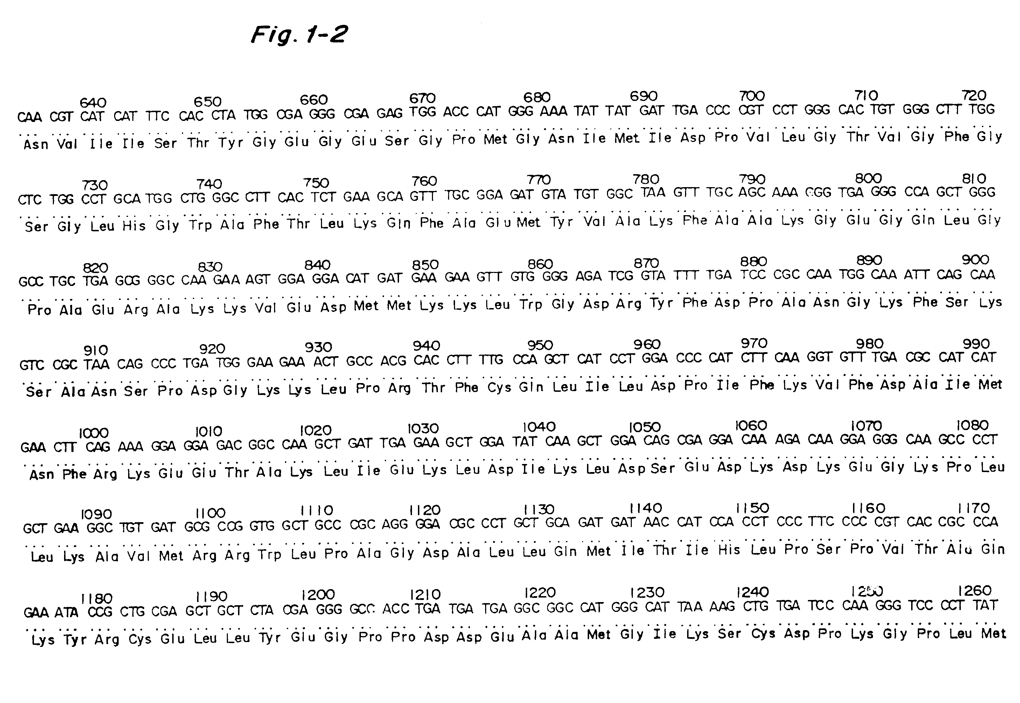

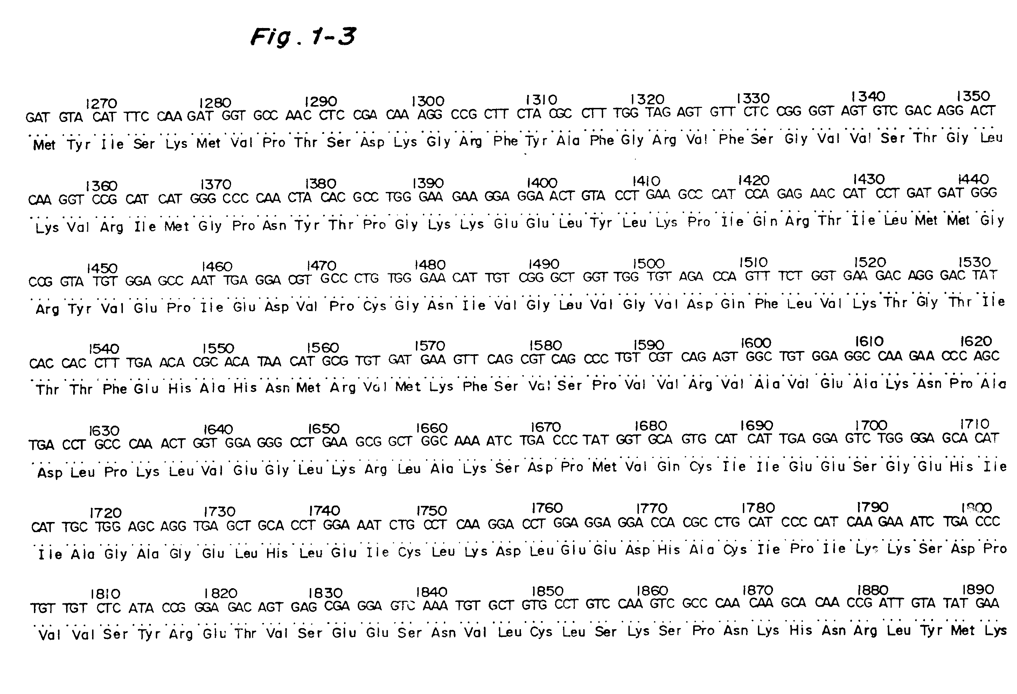

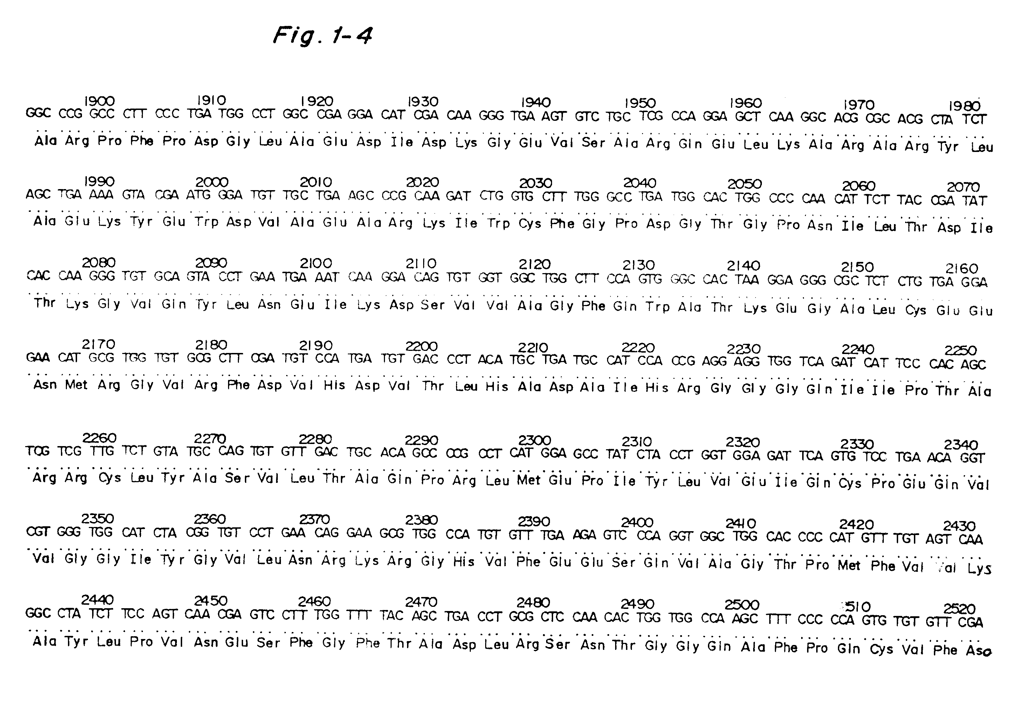

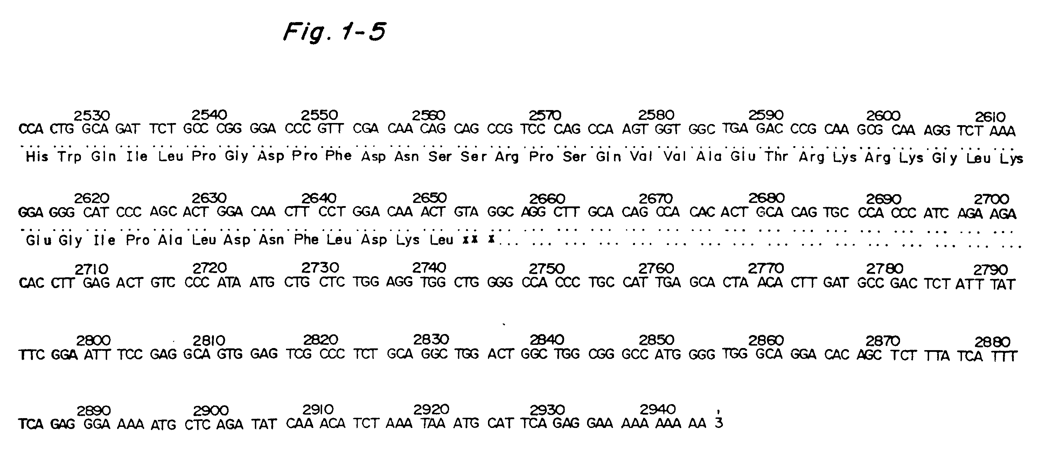

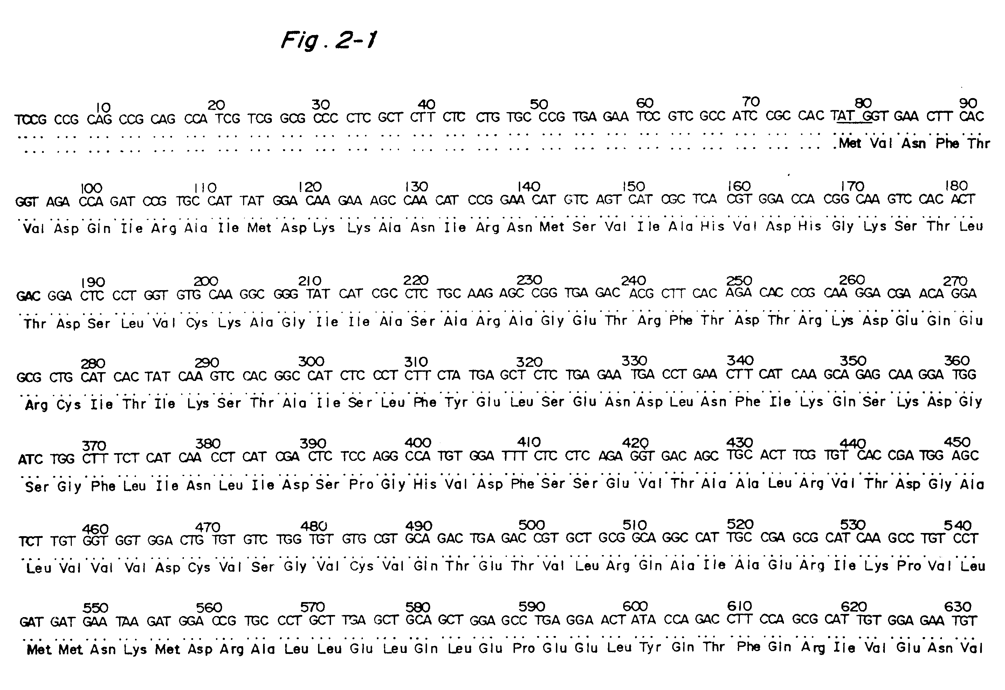

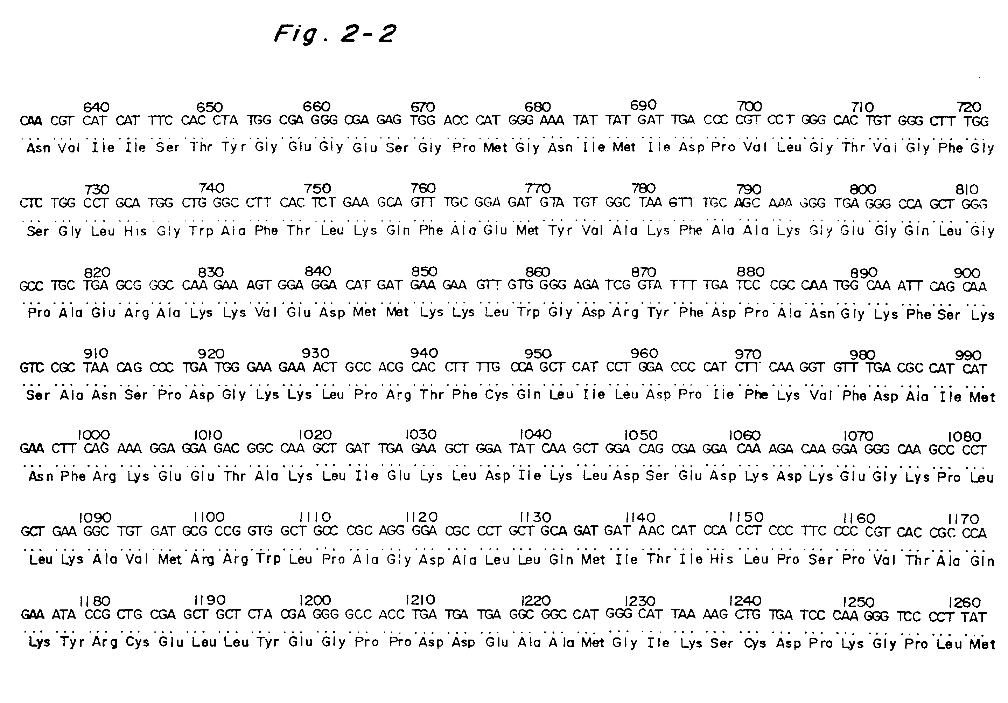

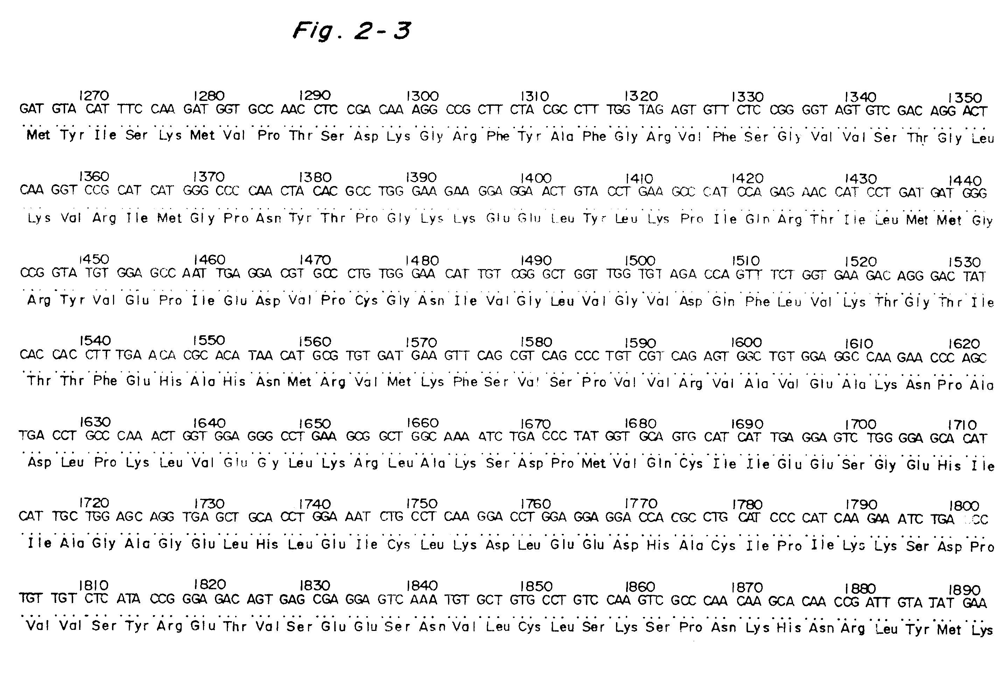

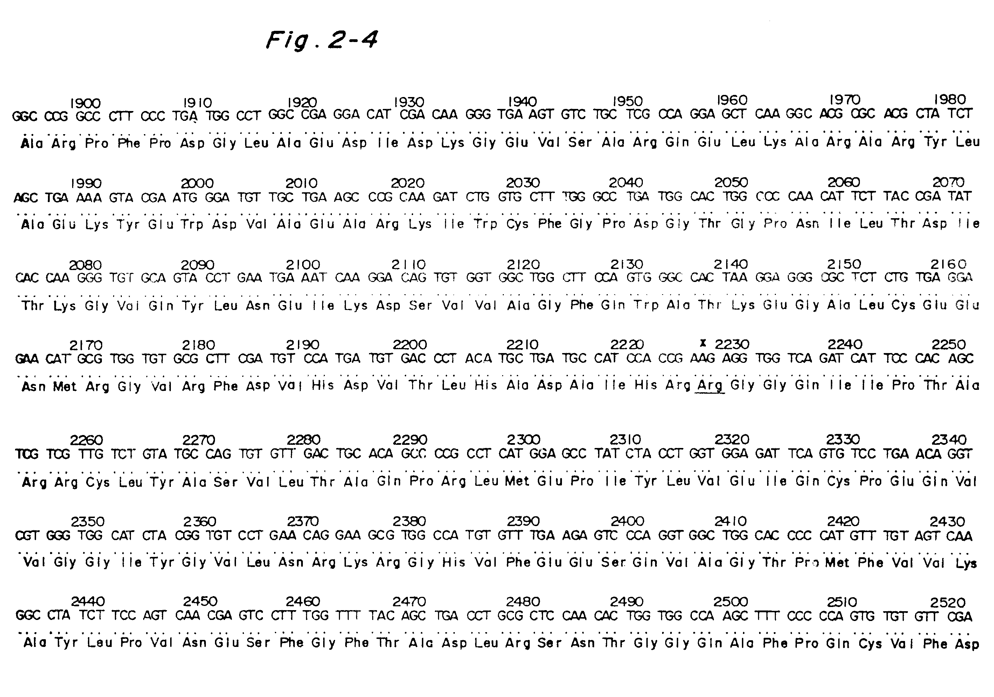

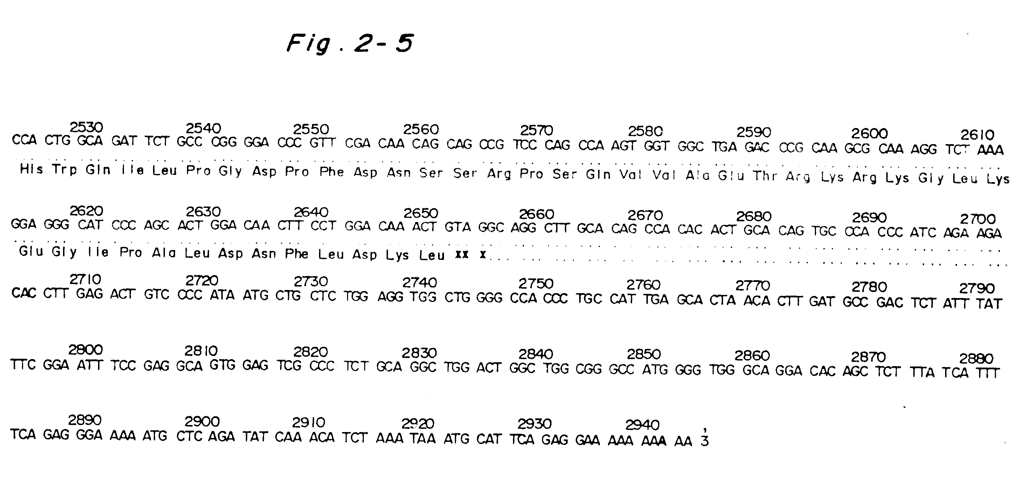

The DNA encoding the naturally occurring EF-2 protein can be prepared by the process consisting of the following steps:
1) preparing mRNA from CHO cells according to conventional procedures;
2) preparing a cDNA library by the use of the Okayama-Berg expression vector for animal cells;
3) transforming E. coli with the resultant expression vector;
4) conducting colony hybridization using an appropriate probe, such as a fragment of a DNA which encodes rat EF-2 or a synthetic oligonucleotide prepared on the basis of the amino acid sequence depicted in Figs. 1 or 2;
5) Selecting clones containing the relevant cDNA; and
6) cloning the cDNA in an appropriate cloning vector.

The variant DNA of the invention can be prepared in the similar manner as mentioned above except that CHO cells resistant to diphtheria toxin or P. aeruginosa exotoxin is employed rather than normal CHO cells.

The following detailed examples are presented by was of illustration of certain specific embodiments of the invention. The examples are representative only and should not be construed as limiting in any respect.

### Example 1

### Preparation of cDNA encoding variant EF-2 which confers resistance to diphtheria toxin

### (1) Synthesis of cDNA

In accordance with the Guanidine-Thiocyanate method (J.M. Chirgwin, A. E. Przybyla, R. J. MacDonald, W. J. Rutter, Biochemistry, 18 5294-5299, 1979), mRNA was prepared from KEE 1 cells resistant to diphtheria toxin, which was derived from CHO. Isolation and characteristic properties of the KEE 1 cells are disclosed in the following reference: K. Kohno, T. Uchida, E. Mekada and Y. Okada, Somat. Cell Molec. Gene, 11 p421-431, 1985.

### (2) Addition of oligo dC chain

The mRNA-containing solution obtained above (44 µl) was added to a reaction mix (220 µl) supplemented with terminal deoxynucleotidyl transferase (40 units, Takara Shuzo), said reaction mix comprising 280 mM sodium cacodylate, 60 mM Tris, pH 6.8, 2mM dithiothreitol, 2mM dCTP, 20 mM CoCl₂, 2.2 µg poly A and 55 µCi ³²P-dCTP (3000 Ci/mmol, Amersham). The mixture was allowed to react at 36°C for 2 minutes, whereby 10 to 15 dC nucleotides were attached to 3'-terminal of the primer cDNA. The reaction was terminated by addition of 0.5 M EDTA (9 µl) and 10% SDS (4.5 µl). After phenol extraction and ethanol precipitation, dC-tailed primer cDNA was recovered and dissolved in distilled water (50 µl).

### (3) Digestion with HindIII

To the aqueous solution obtained above (20 µl) were added 10 fold concentrated HindIII reaction mix (3 µl) and HindIII endonuclease (12 units, Takara Shuzo) to bring the total volume up to 30 µl, and the mixture was allowed to react at 37°C for one hour. After phenol extraction and ethanol precipitation, nucleic acids were recovered and dissolved in distilled water (20 µl).

### (4) Cyclization

THe resulting HindIII-digested dC-tailed cDNA primer (0.42 pmol, 17.5 µl) and dG-tailed linker (0.78 pmol) which had been prepared according to the Okayama-Berg method were dissolved in 175 µl of 10 mM Tris-HCl, pH 7.5, 1 mM EDTA, 0.1 M NaCl, and the mixture was heated at 65°C for 4 minutes, allowed to stand at 42°C for 30 minutes, and chilled to 0°C.

### (5) Ligation

The cyclized vector cDNA obtained above (50 µl) was added to a reaction mix containing 20 mM Tris-HCl, pH 7.5, 4 mM MgCl₂, 10 mM ammonium sulfate, 100 mM potassium chloride, 0.1 mM β-NAD, 50 µg/ml calf serum albumin to make a total volume of 500 µl, and the resultant mixture was added with DNA ligase (2.5 units, Takara Syuzo) and allowed to stand overnight at 12°C.

### (6) Substitution of RNA chain to DNA chain

To the resulting solution obtained above (500 µl) were added 2 mM dNTP solution (10 µl) comprising dATP, dTTP dCTP and dGTP, 10 mM β-NAD (5 µl), E. coli DNA ligase (1 unit, Takara Shuzo), ribonuclease H (2.5 units, Takara Shuzo), and E. coli DNA polymerase (15 units, New England Biolabs), and the mixture was allowed to react at 12°C for one hour followed by at 25°C for one hour.

### (7) Cloning of cDNA encoding variant EF-2

In the manner as detailed below, cDNA library was transformed into E. coli strain HB 101, and colony hybridization was conducted using rat EF-2 as a probe to provide a plasmid pHED 51 which harboured about 2.9 kb insert.

### i) Competent cells of E. coli strain HB 101 were conventionally prepared by the CaCl₂ method (M. Mandel & A. Higa, J. Mol. Biol. 53 p154, 1970) as described below.

An inoculum from a colony grown on a plate was cultured at 37°C for 10 hours in LB medium (5 µl) comprising 1% bacto tryptone, 0.5% yeast extract, 1% NaCl, pH 7.5, and 2 ml portion of the culture was inoculated to LB medium (50 ml) and cultivated at 37°C for 2.5 hours. The resulting culture was totally added to LB medium, and cultivation was continued at 30°C with shaking at 200 rotations /minute until an absorbance of 0.3 at 550 nm was obtained. Immediately after cultivation, the culture was chilled on ice-water and centrifuged at 3,000 rpm for 7 minutes to collect the microorganism cells, which was subsequently dissolved in a small volume (∼ 8 ml) of 50 mM CaCl₂-10% glycerol solution previously cooled to 0°C and finally dissolved in fresh 500 ml volume of the same solution. The cells were collected by centrifugation at 3,000 rpm for 7 minutes and resuspended in a 50 mM CaCl₂-20% glycerol solution at 0°C. The suspension was divided in small portions, frozen in dry ice-ethanol, and stored at -80°C in a freezer.

The competent cells thus obtained can provide 1 ∼ 5 x 10⁷ transformant when 1 µg of pBR 322 DNA (form I) was employed.

### ii) Transformation

A polystyrene test tube was charged with the cDNA library solution obtained in the above item (6) (15 µl), a 10 fold concentrated buffer for transformation (10 µl) which comprises 100 mM CaCl₂ and 400 mM MgCl₂, and distilled water (75 µl). To the test tube was added the competent cells prepared above (100 µl), and the tube was left to stand on ice for 20 minutes. The above procedure was repeated and similar 10 tubes were prepared. Each of the test tubes was allowed to stand at room temperature for 10 minutes, added with LB medium (1 ml), cultured at 37°C for one hour, and centrifuged to collect the cells, which was subsequently suspended in LB medium (200 µl). A 100 µl portion of the suspension was dispersed on 20 sheets of nitrocellulose which had been prepared by overlaying nitrocellulose filter (diameter 82 mm, Milipore Co. HATF) on LB medium supplemented with 1.5% agar and ampicillin (50 µg/ml). A total of 70,000 colonies were generated from the 20 sheets of nitrocellulose. A fresh filter was overlayed on each nitrocellulose as a master plate to prepare a couple of replica. After replication, the master plate was incubated at 37°C for about 3 hours and kept in reserve at 4°C. The replicated plate was incubated in the same manner as above, and the filter on the plate was transferred onto LB medium supplemented with chloramphenicol (200 µg/ml) and cultured at 37°C for 20 hours to promote plasmid amplification.

### iii) Colony Hybridization

### a) DNA fixation on nitrocellulose filter

The filter on which plasmids were amplified was subjected to 5 minutes dipping and 3 minutes draining sequentially with 0.5 N NaOH, 1 M Tris, pH 7.5, and 1 M Tris, pH 7.5, plus 1.5 M NaCl, and finally air-dried for one hour. The filter was then baked at 75°C for 2 hours to fix DNA on the filter.

### b) Pre-hybridization

The filter was washed with each 3 x SSC (SSC: 0.3 M NaCl, 0.03 M sodium citrate) and 0.1% SDS solution at 60°C for 15 minutes, subsequently immersed in a solution comprising 3 x SSC, 10 x Denhardt (50 x Denhardt solution: 1% Ficoll, 1% polyvinylpyrrolidone, 1% calf serum albumin) and a denatured herring sperm DNA, and heated at 60°C for more than 3 hours.

### iv) Preparation of probe by nick translation

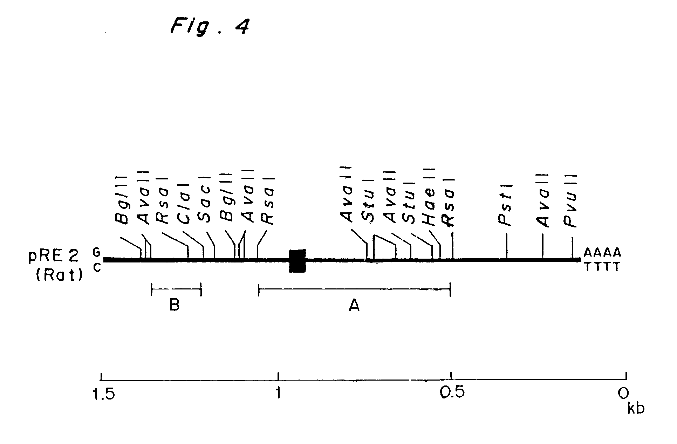

A 576 bp RsaI fragment excised from cDNA coding for rat EF-2 (pRE 2) (Probe A) and a ClaI-AvaII fragment of 161 bp (Probe B, see Fig. 4) were each labeled through conventional nick translation (Rigby, P. W. J., M. Dieckmann, C. Rhodes and P. Berg; J. Mol. Biol. 113 p237, 1977) to obtain probes which have a specific activity of ∼ 10⁸ cpm/µg DNA.

### v) Hybridization

Forty sheets of filter were equally divided to two groups, and each group was immersed in the same solution (15 ml) as in pre-hybridization. To the solution was added Probe A or Probe B which had been denatured by heating at 90°C for 10 minutes followed by rapid cooling, and the mixture was allowed to react at 60°C for 30 hours. The filters were thoroughly washed with 6 x SSC plus 0.03% SDS solution. Autoradiography was conducted on the dried filters and colonies which showed positive reaction to both probes were picked up from the master plates.

### vi) Preparation of plasmid by small scale cultivation

Each of the strains obtained above was grown overnight in LB medium (2 ml), and a plasmid was prepared by the alkaline lysis method (Birnboim, H. C. and J. Doly; Nucleic Acids, Res. 7 p1513, 1979). The plasmid was digested with BamHI or XhoI, and electrophoresed on 1% agarose gel to obtain plasmid pHED51 which carried about 2.9 kb insert.

### (8) Sequencing strategy of cDNA

A BglII-SmaI fragment from the plasmid pHED51 was inserted into mp 18 and mp 19 phages, and the base sequence of the fragment was determined by the method herein after described in Example 2 and compared with that of wild-type origin. This revealed that the BglII-SmaI fragment underwent GC to AT transition by the single point mutation of GC pair located proximal to the afore-mentioned particular amino acid residue, i.e., so-called "diphthamide", which is susceptible to ADP ribosylation. Such transition causes the change in amino acid sequence of His-Arg-Gly to His-Arg-Arg, and such change appears responsible for aquirement of the resistance to diphtheria toxin or P. aeruginosa exotoxin.

### Example 2

### Preparation of cDNA encoding wild-type EF-2

### (1) Preparation of hamster cDNA library

In accordance with the Guanidine Thiocyanate method, total RNA was extracted from CHO K1 cells at logarithmic phase, and mRNA was recovered by oligo dT cellulose column chromatography. In the same manner as in Example 1, a cDNA library was then prepared using the Okayama-Berg expression vector for mammalian cultured cells, as described below.

The mRNA was subjected to thermal denaturation at 65°C for 5 minutes and annealing procedure in the presence of a primer. The resultant hybrid was treated with a reverse transcriptase (Life Science) to allow cDNA synthesis. After dC chain was generated at 3' terminal of the resultant cDNA by the action of a terminal transferase (Takara Shuzo), the cDNA was digested with HindIII.

The HindIII-digested cDNA and dG-tailed linker were cyclized and ligated in the presence of ligase (Takara Shuzo). The ligated product was treated with E. coli DNA polymerase I, ribonuclease H (New England Biolabs), and E. coli ligase to obtain a double stranded cDNA.

### (2) Investigation of hamster cDNA library

In the same manner as in Example 1, the cDNA library obtained above was transformed into E. coli strain HB 101 originated from E. coli strain K12 and about 70,000 colonies were investigated by the use of probe A (Fig. 4), which had been labeled by nick translation. Sixty two positive clones were thus obtained.

The cells carrying desired plasmid were cultured in a small scale, and the plasmids were prepared by the alkaline lysis method. The plasmids were digested with BamHI and XhoI restriction enzymes to determine the size of cDNA inserted into the plasmid. This revealed that a plasmid was about 3 kb in length and contained cDNA corresponding to almost full-length of mRNA for EF-2. This plasmid was designated pHEW 1. E. coli containing this plasmid was designated E. coli HB 101/pHEW 1 accordingly.

### (3) Restriction map of cDNA encoding EF-2

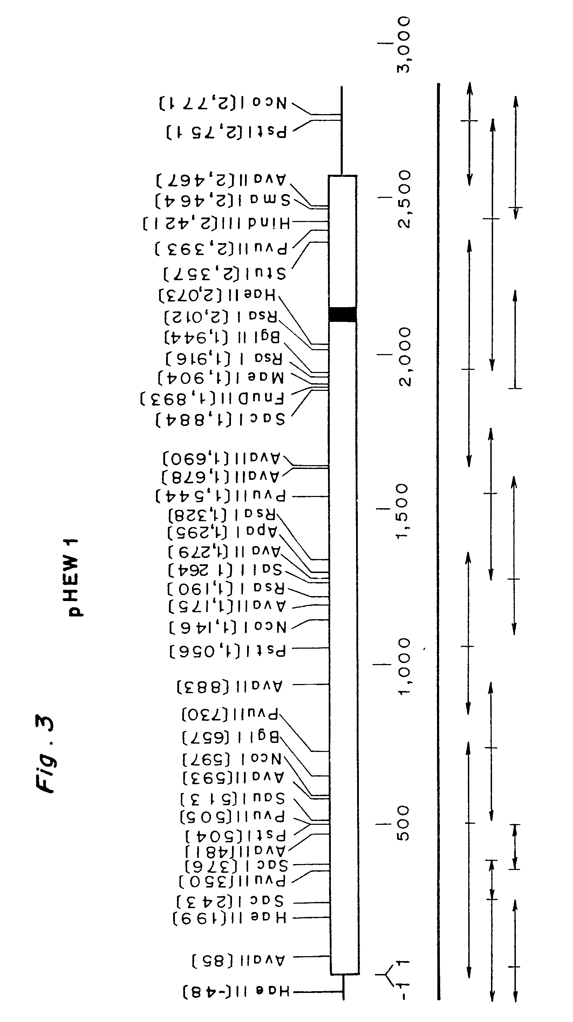

The plasmid pHEW 1 was digested with one or two restriction enzymes, which recognize 6 base pairs, selected from BamHI, XhoI PstI, PvuI, SacI, HindIII, SmaI, BglII, SalI, EcoRI, KpnI, ClaI, AhaIII, HpaI and the like and a restriction map was prepared as shown in Fig. 3 in the accompanying drawings.

### (4) Base sequence of cDNA encoding EF-2

The plasmid pHEW 1 was digested with appropriate enzymes to generate fragments having 100 to 500 base pairs. The fragments were separated on polyacrylamide gel and recloned into multi cloning site of mp 18 or mp 19 derived from M13 phage (See Messing, J., Methods in Enzymology, 101 20-78(1983); Yanisch-Perron, C., Vieira, J. and Messing, J., Gene, 33 103-119(1985)). The mp 18 or mp 19 of RF1 (covalently closed circular form of DNA) was digested with a suitable restriction enzyme, and a fragment to be cloned was inserted into the restriction site. The plasmid was transfected into E. coli strain JM 103. The cells which acquired the fragment were selected by formation of white plaque which reflects the inhibition of synthesis of β-galactosidase in the presence of IPTG. The fragment-containing phage obtained from the plaque was cultivated. A single stranded phage DNA was prepared from the supernatant and used as a template for deoxyribonucleotide synthesis.

A base sequence of the plasmid pHEW 1 and amino acid sequence deduced therefrom are shown in Fig. 1 of the accompanying drawings.

The cDNA encoding wild-type EF-2 harboured in the plasmid pHEW 1 consists of 2,933 base pairs excluding poly A portion, which appears to cover almost all of mRNA sequence.

The EF-2 protein is encoded by 2,574 base pairs raging from start codon ATG at 78 position to stop codon TAG at 2,652 and consists of 857 amino acid residues excluding the initiation methionine.

The amino acid sequence of hamster EF-2 was identical with that of rat EF-2 of which 15 amino acid residues involved in ADP-ribosylation by diphtheria toxin, N-terminal 19 amino acid residues and C-terminal amino acid residue had previously been determined.

### Example 3

### Preparation of cDNA which has a single base substitution and encodes variant EF-2 protein resistant to diphtheria toxin

Plasmids pHEW 1 and pHED 51 were each digested with BglII and SmaI. The resultant larger fragment derived from pHEW 1 and BglII-SmaI fragment (520 bp) from pHED 51 were recombined using ligase to obtain a plasmid, designated pHED 1, in which BglII-SmaI fragment of pHEW 1 was solely replaced by the corresponding gene derived from diphtheria toxin resistant cells. The base sequence of pHED 1 and amino acid sequence deduced therefrom are depicted in Fig. 2 of the accompanying drawings. In cDNA thus obtained a single base of the gene encoding EF-2 has been substituted. E. coli which contains the plasmid pHED 1 was designated E. coli HB 101/pHED 1.

### Reference Example

### Preparation of cDNA encoding rat EF-2

### (1) synthesis of oligonucleotide

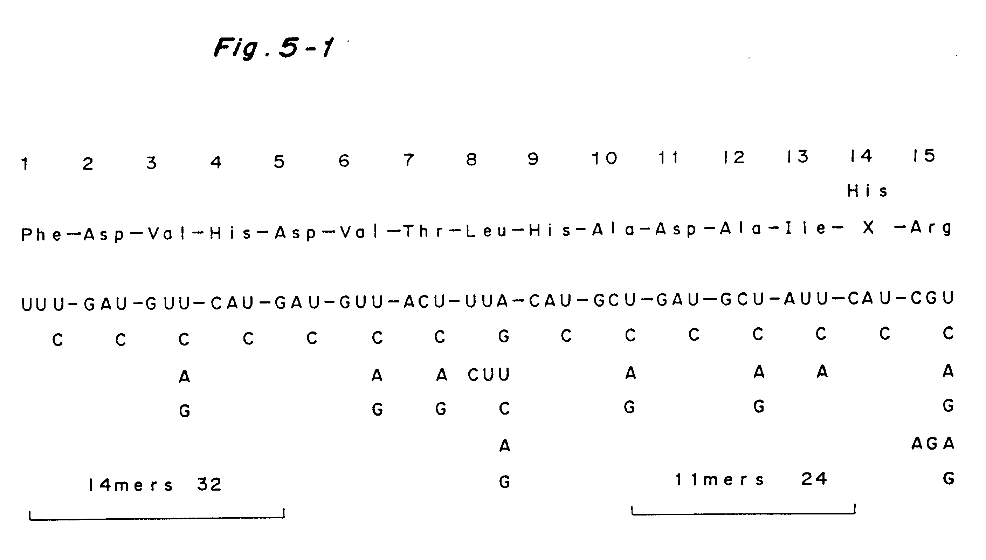

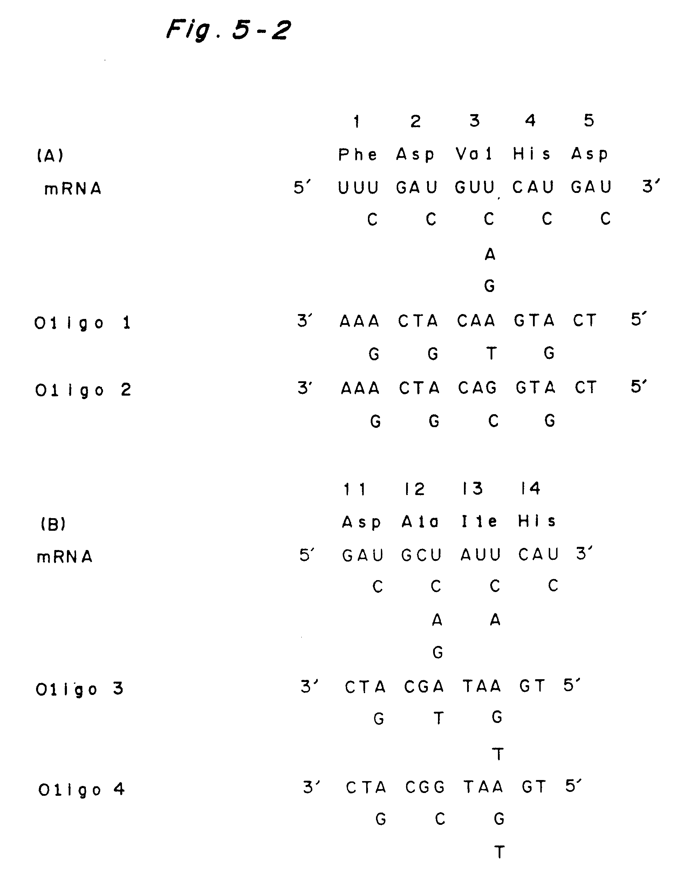

Thirty two 14 mers encoding the amino acid sequence ranging from ¹Phe to ⁵Asp and twenty four 11 mers encoding the sequence covering ¹¹Asp to ¹⁴His, both shown in Fig. 5, were synthesized and used as a probe for cloning cDNA, said 14 mers and 11 mers corresponding mRNA oligonucleotide which encodes a part of known 15 amino acid sequence of rat EF-2 protein including the origin of "diphthamide" (see E. A. Robinson, O. Henriksen, and E. S. Maxwell; J. Biol. Chem., 249 p5088-5093 1974. See also Fig. 5 of the accompanying drawings).

### (2) Screening of rat cDNA library

Using mRNA derived from a rat liver, a cDNA library was prepared in accordance with the method described by H. Okayama and P. Berg, Mol. Cell. Biol., 3 280-289 1983, by the use of the Okayama-Berg cloning vector (ibid., 2 161-170 1982).

The cDNA encoding rat EF-2 was screened using as a probe a synthetic oligonucleotide, Oligo 1 or Oligo 2, labeled with ³²P at 5' terminus. The probes are shown in Fig. 5 of the accompanying drawings. Five positive clones were obtained from about 50,000 colonies. It is confirmed that one clone, pRE 2, encodes rat EF-2 cDNA from Southern blot hybridization analysis and DNA sequencing of pRE 2 plasmid. A restriction map of the plasmid pRE 2 is shown in Fig. 4 of the accompanying drawings.

### (3) Comparison of rat mRNA encoding EF-2 expression with that of hamster

For the purpose of determining the size of mRNA which encodes EF-2 and homology between base sequences of mRANs for rat and hamster EF-2, there were prepared mRNA derived from a rat liver, mRNA derived from CHO-K1, and mRNA derived from CHO-K1 resistant to diphtheria toxin by the Guanidine Thiocyanate method. The resultant mRNAs were subjected to Northern Blotting using, as a probe, RsaI-digested fragment of pRE 2 (probe A, Fig 4). The test revealed that both mRNAs for rat and hamster EF-2 were about 3 kb in length and had high homogeneity to each other.

### Example 4

### Expression of pHED 1 in animal cells

### (1) Expression in mouse L cells

Mouse L cells (8 x 10⁴) were plated on a plastic Petri dish for cultivation (35 mm diameter) and grown at 37°C for 24 hours in a CO₂-incubator. On the other hand, plasmids pHED 1 and pHEW 1 and herring sperm DNA were prepared by calcium phosphate co-precipitation as taught by D. A. Spandidos & N. M. Wilkie, "Transcription and Translation", LPL Press Ltd., edited by B. D. Hames & S. J. Higgins, 1-48, 1984.

The mouse cells were transfected with each of 170 µl of the above DNAs, incubated for 24 hours to facilitate transformation, intensively washed with a fresh culture medium and transferred to a growth medium (αMEM (See Stanners, C.P., Eliceiri, G. L. and Green, H., Nature New Biol., 230 52 (1971)) + 8% FCS (Fetal Calf Serum)). After 24 hours incubation, P. aeruginosa exotoxin A (PA), which exhibits similar toxicity as diphtheria toxin, was added to the medium to make final concentration of 0.1 to 0.5 µg/ml, and cultivation was continued. After 1, 2, 3 and 7 days, the culture was inoculated into F 12 (See Ham, R. G., Proc. Natl. Acad. Sci. USA, 53, 288(1965)) + 10% FCS medium containing 2 µCi/ml of [³H]-leucine and cultured for 2 hours. Respective cultures thus obtained were investigated for protein synthesis, which indicated that the cells which acquired pHEW 1 or herring sperm DNA produced less proteins due to the inhibition of protein synthesis, whereas the cells which acquired pHED 1 exhibited a normal production of proteins.

### (2) Expression of the defined DNAs in several cell lines

In the same manner as described above, the plasmids pHEW 1 and pHED 1 were transformed into Cos-1 cell derived from monkey CV 1, CHO cell, human fibroblast diploid cell, FL cell and HeLa cell, and expression of resistance against the toxin was examined for each cell on the basis of protein synthesis. The experimental results are shown in Table 1, which demonstrates that pHED 1, unlike pHEW 1, more or less endows every cell with resistance to the toxin.

**Table 1**

| Expression of toxin resistance in various cell lines | | | |
|---|---|---|---|
| Cell lines | toxin(µg/ml) | DNA introduced | |
| | | pHED 1 | pHEW 1 |
| | | Incorporation of [³H]Leucine(CPM/plate) *a | |
| mouse L | PA 0.1 | 8520 | 300 |
| monkey Cos-1 | DT 10⁻⁴ | 1510 | 230 |
| human HEL | DT 10⁻³(*C) | 2280 | 290 |
| human FL | DT 10⁻⁴ | 1270 | 580 |
| hamster CHO-KI(*b) | DT 10⁻³ | 2380 | 1300 |

| | | | |
|---|---|---|---|
| *a Cells were plated at ratio of 5 to 10 x 10⁴ cells/35 mm dish, transformed with plasmid by calcium phosphate co-precipitation in conventional manner, incubated for 24 hours for expression, and added with the toxin. After 2 days, the cells were incubated in the presence of 2 µl/ml of [³H]leucine, and protein synthesis was quantitatively determined by radioassay. | | | |
| *b CHO cells were tested one day after addition of the toxin. | | | |
| *c Twenty-four hours after addition of diphtheria toxin, the cells were cultured in the absence of the toxin for 24 hours. | | | |

### (3) Long term transformation

Mouse L cells were plated on a Petri dish in a ratio of 5 x 10⁵ cells/100 mm, cultured for 24 hours, and transfected with each of pHED 1 and pHEW 1 by the use of calcium phosphate co-precipitation as described above.

After 24 hours of transfection, the culture medium was removed and the cells were thoroughly washed with fresh culture medium. The cells were subsequently cultivated in fresh culture medium for 24 hours and, after addition of PA at final concentration of 0.05 µg/ml, cultivation was continued for 3 weeks in the presence of the toxin, and the number of the resulting colonies was counted. The cells transfected with pHEW 1 gave no detectable colony, whereas those transfected with pHED 1 yielded about 80 colonies per plate. Table 2 below presents details of the colony formation.

**Table 2**

| Colony formation on toxin-containing medium | | |
|---|---|---|
| DNA | Number of Colony/100 mm dish | |
| pHED 1 | 77 | 78 (average) |
| | 96 | |
| | 87 | |
| | 84 | |
| | 45 | |
| pHEW 1 | 0 | 0 (average) |
| | 0 | |
| | 0 | |
| | 0 | |
| | 0 | |

LTK⁻ cells were plated at ratio of 5 x 10⁵ cells/100 mm dish and transfected with plasmid by calcium phosphate co-precipitation in conventional manner. After cultivation in the presence of 0.05 µg/ml of PA for 3 weeks, colony formation was observed.

### (4) Co-transformation with TK gene and pHED 1

Mouse LTK⁻ cells were plated on a Petri dish at ratio of 5 x 10⁵ cells/100 mm and incubated for 24 hours. Subsequently, pHED 1 and herpes simplex virus TK gene were transformed into the cells by calcium phosphate co-precipitation at the final concentration of 10 µg/ml. Following 24 hours-cultivation, the cells were washed, transferred to a fresh growth medium, and incubated for 24 hours. The culture was then added with PA at the final concentration of 0.1 µg/ml and cultivated for additional 3 weeks allowing to form colonies. The culture medium was changed to HAT medium, and the magnitude of incorporation and expression of the TK gene was investigated revealing that 20 to 30% of the resultant colonies expressed TK gene.

## Claims

1. A DNA sequence encoding elongation factor 2, as defined by the base sequence depicted in Fig. 1.

2. A DNA sequence encoding a variant elongation factor 2 which is resistant to diphtheria toxin or *Pseudomonas aeruginosa* exotoxin, obtainable by
(1) preparing mRNA from CHO cells resistant to diphtheria toxin or *Pseudomonas aeruginosa* exotoxin,
(2) preparing a cDNA library by the use of an expression vector,
(3) transforming E. coli with the resultant expression vector,
(4) conducting colony hybridization using a synthetic oligonucleotide prepared on the basis of the sequences depicted in Fig. 1 or 2,
(5) selecting clones containing the relevant cDNA, and
(6) cloning the cDNA in an appropriate cloning vector,
wherein said elongation factor 2 has a change in its amino acid sequence based on a point mutation of the coding region, said change being responsible for the resistance to diphtheria toxin or Pseudomonas aeruginosa exotoxin.

3. The DNA sequence according to claim 2, as defined by the base sequence depicted in Fig. 2.

4. A replicable plasmid vector capable, in the transformed microorganism or animal cell culture, of expressing the DNA sequence according to any one of claims 1 to 3.

5. A microorganism or animal cell culture transformed with the replicable plasmid vector according to claim 4.

6. A method for selecting cells transformed with an exogenous DNA sequence from untransformed cells which comprises subjecting cells to transformation with an exogenous DNA sequence containing the DNA sequence according to claim 2 or 3, culturing the cells in the presence of the diphtheria toxin and/or *Pseudomonas aeruginosa* exotoxin, and isolating the cells remaining alive.

7. The method according to claim 6, wherein the exogenous DNA sequence further contains a gene of interest ligated to the DNA sequence.

8. Use of the DNA sequence according to claim 2 or 3 as a selective marker.

## Patentansprüche

1. DNA-Sequenz gemäß der Definition der Basensequenz in Fig. 1, die den Elongationsfaktor 2 codiert.

2. DNA-Sequenz, die eine Elongationsfaktor-2-Variante codiert, welche resistent gegenüber Diphtherie-Toxin oder Pseudomonas aeruginosa-Exotoxin ist, erhältlich durch
(1) Herstellung von mRNA aus CHO-Zellen, die resistent gegenüber Diphtherie-Toxin oder Pseudomonas aeruginosa-Exotoxin sind,
(2) Herstellung einer cDNA-Bank durch Verwendung eines Expressionsvektors,
(3) Transformierung von E. coli mit dem erhaltenen Expressionsvektor,
(4) Durchführung einer Koloniehybridisierung unter Verwendung eines synthetischen Oligonucleotids, das auf der Grundlage der in den Figuren 1 oder 2 angegebenen Sequenzen hergestellt wurde,
(5) Auswahl von Clonen, die die relevante cDNA enthalten, und
(6) Clonierung der cDNA in einem geeigneten Clonierungsvektor,
wobei der Elongationsfaktor 2 eine Änderung in seiner Aminosäuresequenz aufweist, die durch eine Punktmutation der Codierungsregion verursacht ist, wobei diese Änderung für die Resistenz gegenüber Diphtherie-Toxin oder Pseudomomonas aeruginosa-Exotoxin verantwortlich ist.

3. DNA-Sequenz nach Anspruch 2, gemäß der Definition der Basensequenz in Figur 2.

4. Replizierbarer Plasmidvektor, der fähig ist, in dem transformierten Mikroorganismus oder der transformierten tierischen Zellkultur die DNA-Sequenz gemäß einem der Ansprüche 1 bis 3 zu exprimieren.

5. Mikroorganismus oder tierische Zellkultur, die mit dem replizierbaren Plasmidvektor nach Anspruch 4 transformiert ist.

6. Verfahren zur Auswahl von Zellen, die mit einer exogenen DNA-Sequenz transformiert sind, von untransformierten Zellen, umfassend die Durchführung einer Transformation mit einer exogenen DNA-Sequenz, die die DNA-Sequenz nach Anspruch 2 oder 3 enthält, Züchten der Zellen in Gegenwart von Diphtherie-Toxin und/oder Pseudomonas aeruginosa-Exotoxin, und Isolierung der am Leben bleibenden Zellen.

7. Verfahren nach Anspruch 6, wobei die exogene DNA-Sequenz weiterhin ein Gen von Interesse enthält, das an die DNA-Sequenz gebunden ist.

8. Verwendung der DNA-Sequenz nach Anspruch 2 oder 3 als selektiver Marker.

## Revendications

1. Séquence d'ADN codant pour le facteur d'élongation 2, telle qu'elle est définie par la séquence de bases représentée sur la figure 1.

2. Séquence d'ADN codant pour une variante du facteur d'élongation 2 qui est résistante à la toxine diphtérique ou à l'exotoxine de Pseudomonas aeruginosa, que l'on peut obtenir
(1) en préparant un ARNm de cellules CHO résistantes à la toxine diphtérique ou à l'exotoxine de Pseudomonas aeruginosa,
(2) en préparant une banque d'ADNc à l'aide d'un vecteur d'expression,
(3) en transformant E. Coli avec le vecteur d'expression résultant,
(4) en réalisant une hybridation sur colonie à l'aide d'un oligonucléotide synthétique préparé sur la base des séquences représentées sur la figure 1 ou 2,
(5) en sélectionnant les clones contenant l'ADNc pertinent, et
(6) en clonant l'ADNc dans un vecteur de clonage approprié,
ledit facteur d'élongation 2 présentant une modification de sa séquence d'acides aminés basée sur une mutation ponctuelle de la région codante, ladite modification étant responsable de la résistance à la toxine diphtérique ou à l'exotoxine de Pseudomonas aeruginosa.

3. Séquence d'ADN selon la revendication 2, telle qu'elle est définie par la séquence de bases représentée sur la figure 2.

4. Vecteur plasmidique réplicable capable, dans le micro-organisme transformé ou dans la culture de cellules animales transformées, d'exprimer la séquence d'ADN selon l'une quelconque des revendications 1 à 3.

5. Micro-organisme transformé ou culture de cellules animales transformées avec le vecteur plasmidique réplicable selon la revendication 4.

6. Procédé de sélection de cellules transformées avec une séquence d'ADN exogène parmi des cellules non transformées qui comprend la soumission des cellules à une transformation avec une séquence d'ADN exogène contenant la séquence d'ADN selon la revendication 2 ou 3, la culture des cellules en présence de la toxine diphtérique et/ou de l'exotoxine de Pseudomonas aeruginosa, et l'isolement des cellules restant vivantes.

7. Procédé selon la revendication 6, dans lequel la séquence d'ADN exogène contient en outre un gène d'intérêt ligaturé à la séquence d'ADN.

8. Utilisation de la séquence d'ADN selon la revendication 2 ou 3 comme marqueur sélectif.
